# EUROPEAN PATENT SPECIFICATION

(11) **EP 2 421 541 B1**
(45) Date of publication and mention of the grant of the patent: **11.07.2018**
(21) Application number: 10714956.9
(22) Date of filing: 23.04.2010
(51) Int. Cl.: A61K 31/7016, A61K 31/702, A61P 29/00

(54) **NOVEL USE**
NEUE ANWENDUNG
NOUVELLE UTILISATION

(30) Priority: 23.04.2009 GB 0906983; 27.11.2009 GB 0920784
(43) Date of publication of application: 29.02.2012
(73) Proprietor: Clasado Inc., Panama (PA)
(72) Inventor: TZORTZIS, Georgios, Berkshire RG2 7RR (GB); VULEVIC, Jelena, Berkshire RG2 7RR (GB); ATTANASIO, Francesco, Berkshire RG2 7PW (GB)
(74) Representative: Dolleymores
(86) International application number: PCT/GB2010/050659
(87) International publication number: WO 2010/122344

(56) References cited:
- WO-A1-2004/052121
- WO-A1-2005/003329
- WO-A1-2010/023422

## Description

The present invention relates to the use of a galactooligosaccharide, in the prevention of inflammation, in particular in the prevention of intestinal inflammation. Galactooligosaccharides are non-digestible carbohydrates, which are resistant to mammalian gastrointestinal digestive enzymes but are fermented by specific colonic bacteria.

The human gut flora comprises pathogenic, benign and beneficial microbial genera. A predominance of the former can lead to intestinal disorders that can be both acute (eg gastroenteritis) and chronic (eg inflammatory bowel disease and some intestinal cancers).

Prebiotics, which are defined as a non-digestible food ingredients that beneficially affect the host by selectively stimulating the growth and/or activity of one or a limited number of bacteria in the colon, thereby resulting in an improvement in the health of the host, have been shown to have an indirect protective effect in a number of inflammatory conditions such as inflammatory bowel disease (IBD). It has been found in some IBD patients that the adaptive immune system is hyper-responsive to commensal intestinal flora (see Guarner F, Malagelada JR, Best Pract. Res. Clin. Gatroenterol., (2003); 17; 793-804). As a result, prebiotics have been used to enhance beneficial gut microflora which has helped to prevent a relapse in the disease (see Sartor RD., Gastroenterology, (2004), 126, 1620-1633).

One group of compounds that is classified as prebiotics are the galactooligosaccharides. These are galactose-containing oligosaccharides of the form Glc β1-4 [Gal β 1-6]ₙ where n = 2 - 5 and are produced from lactose syrup using the transgalactosylase activity of the enzyme β-galactosidase (Crittenden, (1999) Probiotics : A Critical Review, Tannock, G. (ed) Horizon Scientific Press, Wymondham, pp 141-156).

EP 1 644 482 discloses a novel strain of *Bifidobacterium bidifum* that produces a galactosidase enzyme activity that converts lactose to a novel mixture of galactooligosaccharides. This mixture of galactooligosaccharides comprises disaccharides Gal (β 1-3) Glc; Gal (β 1-3)-Gal; Gal (β 1-6)-Gal; Gal (α 1-6)-Gal; trisaccharides Gal (β 1-6)-Gal (β 1-4)-Glc; or Gal (β 1-3)-Gal (β 1-4)-Glc; tetrasaccharide Gal (β 1-6)-Gal (β 1-6)-Gal (β 1-4)-Glc or pentasaccharide Gal (β 1-6)-Gal (β 1-6)-Gal (β 1-6)-Gal (β 1-4)-Glc and has been shown to have prebiotic properties and to increase the population of the beneficial bacteria bifidobacteria and lactobacilli. This mixture of galactooligosaccharides is marketed commercially under the name Bimuno (Registered Trade mark) and is available from Clasado Ltd (Milton Keynes, UK).

Vulevic, J et al described in Am. J Clin. Nutr., (2008), 88 : 1438-46 how administering galactooligosaccharides to healthy elderly persons resulted in positive effects on both the faecal microflora composition and the immune response.

It has now been found that a galactooligosaccharide having a DP (degree of polymerisation) of 3 or more can directly modulate the inflammatory response of the mammalian intestinal mucosa. In particular, it attenuates the pro-inflammatory chemokine response, in the presence of inflammatory agents. Thus, such a galactooligosaccharide may be useful in treating such intestinal inflammatory conditions as inflammatory bowel disease, colitis, entero necrotizing colitis, pseudomembranous colitis, ulcerative colitis, Crohn's disease, diverticulitis, ischemia, etc.

According to the invention there is provided a galactooligosaccharide having a DP of 3 or more which galactooligosaccharide is selected from the group consisting of trisaccharides Gal (β 1-6)- Gal (β 1-4)- Glc, Gal (β 1-3)- Gal (β 1-4)-Glc, tetrasaccharide Gal (β 1-6)- Gal (β 1-6)- Gal (β 1-4)-Glc, and pentasaccharide Gal (β 1-6)- Gal (β 1-6)- Gal (β 1-6)- Gal (β 1-4)-Glc for use in the prevention of intestinal inflammatory disorders. Preferably the galactooligosaccharide has a DP of from 3 to 5.

Enterocytes form a single polarized epithelial layer separating the luminal environment from the host. They are active contributors to the host defence. Their innate immune response to any inflammatory stimuli is primarily responsible for rapidly regenerating the barrier function of the epithelium. The epithelium can be induced to express pro-inflammatory cytokines and chemokines that begin the process of recruiting innate immune cells such as neutrophils to the damaged mucosa, if necessary. For example, pro-inflammatory chemokines, such as IL-8, can be stimulated during an immune response by epithelial cells and by macrophages to recruit neutrophils and PMN's (polymorphonuclear leukocytes) to the inflamed mucosa. Macrophage Inflammatory Protein-3α (MIP-3α) or CCL20 is another chemokine that elicits the adaptive immune system by activating the lymphocytes and dendritic cells through activation of chemokine receptor CCR6. The IL-8 and MIP-3α (CCL20) induction indicates the degree of response to an inflammation challenge.

The effect of the galactooligosaccharide on the inflammatory response of different adult colonic cell culture models has been studied. It was found unexpectedly that at physiologic concentrations, it attenuates the pro-inflammatory chemokine response induced by TNF-α inflammatory stimulus in intestinal epithelial cells, ie human enterocytes.

The galactooligosaccharide may be prepared from the commercially available mixture of galactooligosaccharides known as Bimuno by purification using for example size exclusion chromatography using for example a Biogel P2 column maintained at room temperature. The sample may be prepared by dissolving the powder in water 10% w/v and eluting at a rate of 2 ml/min with deionised water.

Alternatively, the active fraction of the Bimuno mixture may be prepared by enzymatic transgalactosylation using the appropriate enzyme.

The galactooligosaccharide may be presented as a powder, a syrup or in the form of a soft pastille. It may be administered to a patient suffering from an inflammatory disorder, for example an intestinal inflammatory disorder, daily in an effective dose of the active galactooligosaccharide of from 1 to 10g, preferably from 2 to 5g, most preferably 2.75g. This can be taken in one single dose or in two separate doses several hours apart. The galactooligosaccharide powder may be added to a hot drink or sprinkled on food. The syrup can be consumed by itself or alternatively mixed into a beverage or spread on food. The soft pastille is chewed in the mouth.

In order to prevent inflammation the galactooligosaccharide may be administered to an individual in an effective daily dose of 1 to 10g, preferably 2 to 5g, most preferably 2.75g.

The present invention will be further described by way of reference to the following examples and figures.
Figure 1 shows the effect of B-GOS on the TNF-α induced IL-8 secretion in T84 cells;
Figures 2(A) and (B) show the effect of B-GOS on TNF-α induced IL-8 and MIP-3α secretion in NCM-460 cells;
Figures 3(A) and (B) show the effect of B-GOS on the expression of IL-8 and MIP-3α mRNA in TNF-α treated NCM-460 cells;
Figures 4(A), (B) and (C) show the effect of B-GOS on the translocation of NF-κB p65 protein into the nuclei of TNF-α treated NCM-460 cells;
Figures 5 and 6 show the effect of B-GOS on TNF-α induced IL-8 secretion in NCM-460 cells;

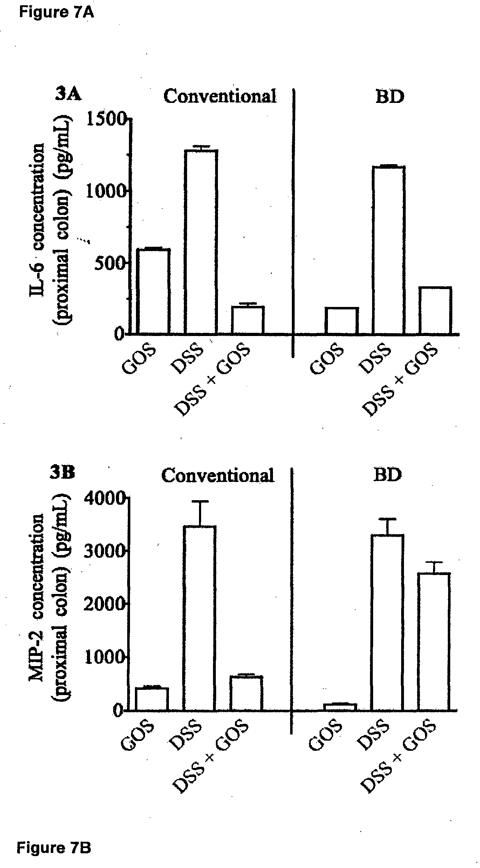

Figures 7(A) and (B) show the effect of B-GOS on IL-6 and MIP-2 secretion in DSS treated mice;
Figures 8(A) and (B) show the effect of different fractions of galactooligosaccharides on the production of IL-8 and MIP-3α respectively; and
Figures 9(A) and (B) show the effect on the production of TNF-α induced IL 8 and MIP-3 α respectively, when NCM460 cells are treated with Bimuno and the DP3 and DP>3 fractions thereof.

### EXAMPLE 1

### Effect of galactooligosaccharides on cytokine secretion

Intestinal epithelial cells were grown to confluence in 24-well plates from an initial concentration of 5x10⁵ cells/mL. When the cells reached 70% confluence, they were treated in quadruplicate as follows: (i) negative control, (ii) TNF-α (10 ng/mL) positive control, (iii) B-GOS (5 g/L) and (iv) TNF-α (10 ng/mL) with B-GOS (5 g/L). A concentration of 5 g/L of oligosaccharides was used since this is the physiological concentration of oligosaccharides found in human milk. After 16 hours, supernatants were collected and stored at -20°C for IL-8 and MIP-3α secretion to be determined later by ELISA. In following experiments, TNF-α was replaced by IL1β or flagellin.

***Quantitation of IL-8.*** The IL-8 concentration was measured by ELISA as described previously (Claud EC, Savidge T, Walker WA 2003 Modulation of human intestinal epithelial cell IL-8 secretion by human milk factors. Pediatr Res 53:419-425). Briefly, each well of a 96-well high bond plate (Nunc Immulon, Fisher Scientific, Middletown, VA, USA) was coated overnight with 100 µL of 3 µg/mL mouse anti-human IL-8 monoclonal antibody, washed three times with 200 µL of 1% BSA in PBS and incubated with 100 µL of samples at 37°C for one hour. The wells were then washed three times and incubated with 100 µL of 0.1 µg/mL biotin-labelled mouse antihuman IL-8 antibody for one hour. After another wash, each well was incubated with 100 µL horseradish peroxidase, washed again before incubating with 100 µL O-phenylenediamine dihydrochloride and hydrogen peroxide. The reaction was stopped with 100 µL 2N H2SO4 and the absorbance was read at 490 nm. The concentration of IL-8 in the samples was calculated from the IL-8 standard curve.

***Quantitation of MIP-3α.*** The amount of MIP-3α secretion was measured by ELISA similar to IL-8, except that the plate was coated overnight with 100 µL 2.0 µg/mL mouse anti-human MIP-3α monoclonal antibody. The detection antibody, biotin-labelled mouse antihuman MIP-3α antibody, was used as detection antibody at a concentration of 50 ng/mL with a volume of 100 µL. The concentration of MIP-3α in the samples was calculated from the MIP-3α standard curve.

***Cell viability assay.*** B-GOS cytotoxicity was investigated using the trypan blue exclusion test. NCM-460 cells were grown on coverslips from an initial concentration of 2x105 cells/mL. The cells were treated in triplicate with: B-GOS (5 g/L) or control medium. After 16 hours, NCM-460 cells were assayed for cell viability by trypan blue exclusion assay (Raimondi F, Crivaro V, Capasso L, Maiuri L, Santoro P, Tucci M, Barone MV, Pappacoda S, Paludetto R 2006 Unconjugated bilirubin modulates the intestinal epithelial barrier function in a human-derived in vitro model. Pediatr Res 60:30-33)*.* There was no significant effect of B-GOS on the viability of the cells at this concentration.

***Effect of B-GOS on induction of cytokine transcription.*** NCM-460 cells were grown to confluence in 6-well plates from an initial concentration of 5x105 cells/mL. When the cells reached 70% confluence, they were treated as follows in quadruplicate: (i) negative control, (ii) TNF-α or IL1β or flagellin (10 ng/mL) positive control, and (iii) TNF-α or IL1β or flagellin (10 ng/mL) with B-GOS (5 g/L). After 18 hours, total cellular RNA was isolated by Trizol-chloroform extraction. Using the SuperScript III Platinum SYBR Green One-Step qRT-PCR kit, the mRNA expression of IL-8, MIP-3α and MCP-1 was measured on a MJ Opticon 2 and standardized to mRNA expression of GAPDH.

***Effect of B-GOS on NF-κB translocation.*** NCM-460 cells were grown to 70% confluency on cover slips and treated in duplicate for 10 or 30 minutes as follows: (i) negative control, (ii) TNF-α (10 ng/mL) positive control, and (iii) TNF-α (10 ng/mL) with B-GOS (5 g/L). The medium was removed and the cells were fixed in 4% paraformaldehyde. After permeabilization with methanol and blocking with 10% goat serum in 0.25% BSA in TBS, the cells were probed with rabbit anti-human NF-κB p65 polyclonal antibody. After washing, the cells were incubated with CyTM 3-conjugated goat anti-rabbit antibody. The cover slips were then washed and mounted on a glass slide to be visualized under the microscope (Nikon Eclipse TE2000-S).

### Materials

TNF-α cytokine, IL1β, flagellin, streptavidin-HRP and human CCL20-MIP-3α ELISA development kits (Quantikine) were obtained from R&D Systems (Minneapolis, MN, USA). Antihuman IL-8 and mouse anti-human IL-8 antibodies were obtained from Pierce Endogen (Woburn, MA, USA). O-phenylenediamine tablets were obtained from Pierce (Rockford, IL, USA). Trizol, SuperScript III Platinum SYBR Green One-Step qRT-PCR kits and other reagents necessary for qRT-PCR were obtained from Invitrogen (Carlsbad, CA, USA). DMEM/F12 medium, CMRL medium, penicillin, streptomycin and Hepes buffer were obtained from Gibco-Invitrogen (Carlsbad, CA, USA). Fetal bovine serum was obtained from Atlanta Biologicals (Lawrenceville, GA, USA). M3D was obtained from Incell Corp. (San Antonio, TX, USA). Rabbit anti-human NF-κB (p65) polyclonal antibody was obtained from Calbiochem (Gibbstown, NJ, USA). CyTM 3-conjugated F(ab')2 fragment goat anti-rabbit IgG was obtained from Jackson ImmunoResearch (West Grove, PA, USA). All other reagents for immunofluorescence were obtained from Vector Lab (Burlingame, CA, USA). All other reagents were of analytical or molecular biological grade from Sigma-Aldrich (St. Louis, MO, USA).

***B-Galacto-oligosaccharides B-GOS.*** Bimuno® was supplied by Clasado Ltd., Milton Keynes, UK.

***Intestinal Epithelial Cell lines.*** Two adult human intestinal epithelial culture models were used in these studies: T84 and NCM-460 cells are transformed and untransformed colonic epithelial cells, respectively. Cells were cultured in Falcon cell culture dishes at 37°C with 95% O2 and 5% CO2 atmosphere saturated with water vapour. T84 culture medium consisted of DMEM/F12 supplemented with FBS (5%), Hepes buffer, glutamine, non-essential amino acids, penicillin and streptomycin (12). NCM-460 culture medium consisted of M3D medium supplemented with FBS (10%), penicillin and streptomycin as described previously (13).

***Statistical analysis.*** Induction of cytokines was standardized to the positive control with error bars representing standard error (SE). Comparisons between groups were performed using a two-tailed Student's t test. Gene expression data obtained by qRT-PCR was expressed as the mean with SE. Comparisons between groups were performed using a Student's two-tailed t test after logarithmic transformation. A *p* value <0.05 was considered statistically significant and indicated by an asterisk (*), a *p* value <0.01 was indicated by two asterisks (**) and a *p* value <0.001 was indicated by three asterisks (***).

### Results

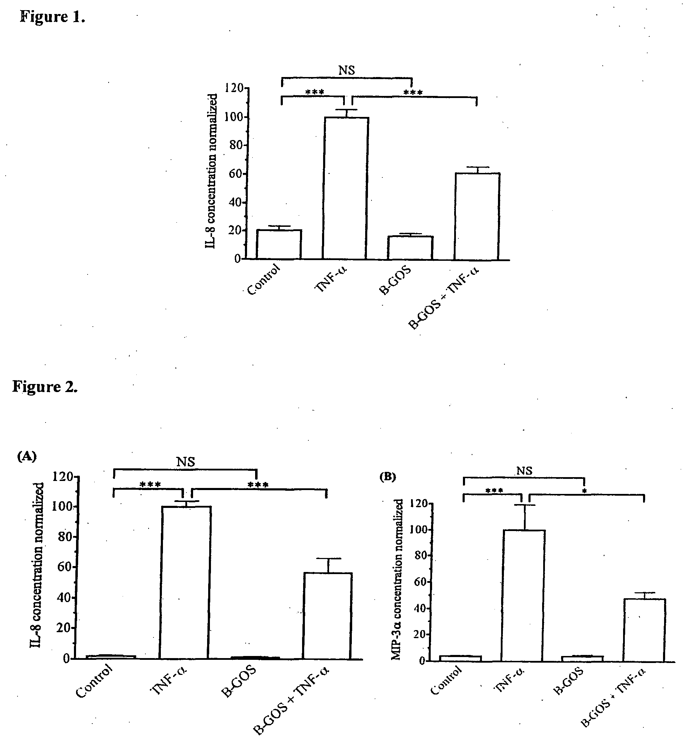

### Effect of galactooligosaccharides B-GOS on cytokine secretion in T84 cells (Figure 1).

TNF-α-induced IL-8 secretion in T84 cells was normalized to 100% to allow for comparison between 4 independent experiments. The untreated T84 cells had a basal IL-8 secretion at 20.5%. Upon TNF-α stimulation, IL-8 secretion was significantly increased by 4.9 fold (*p* < 0.001).

To determine the effect of B-GOS, T84 cells were stimulated with or without TNF-α in the presence of galacto-oligosaccharides B-GOS (5 g/L). B-GOS-treated T84 cells secreted IL-8 at 16.4%. This was not significantly different from basal level of untreated T84 cells. Upon stimulation with TNF-α, B-GOS significantly attenuated IL-8 secretion by 38.5% (*p* < 0.001).

### Effect of galactooligosaccharides B-GOS on cytokine secretion in NCM-460 cells (Figure 2, Figure 5, Figure 6).

TNF-α-induced IL-8 and MIP-3α secretion in NCM-460 cells was normalized to 100% to allow for comparison between 4 independent experiments. The untreated NCM-460 cells had a basal IL-8 and MIP-3α secretion at 1.7% and 4.0% respectively. Upon TNF-α stimulation, IL-8 and MIP-3α secretion was significantly increased by 58.8 fold (*p* < 0.001) (Figure 2A) and 25.0 fold (*p* < 0.001) (Figure 2B) respectively.

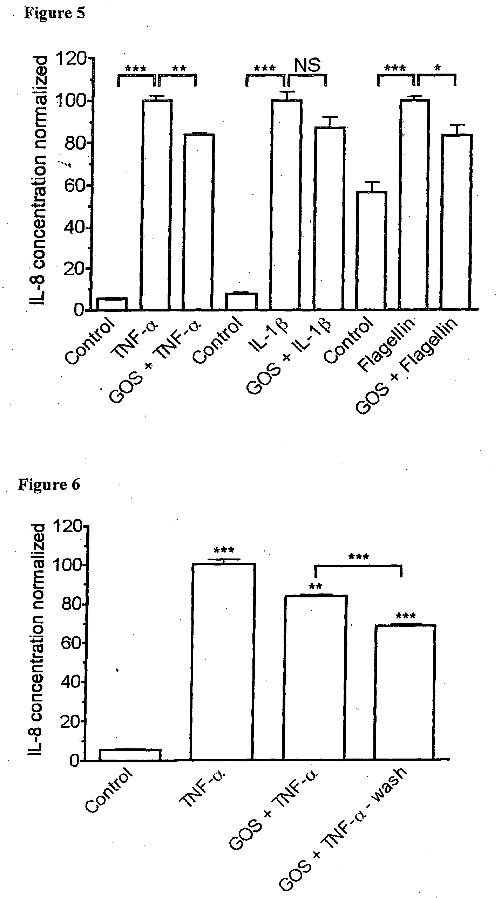

To determine the effect of B-GOS, NCM-460 cells were stimulated with or without TNF-α in the presence of galacto-oligosaccharides B-GOS (5 g/L). B-GOS-treated NCM-460 cells secreted IL-8 and MIP-3α at 1.1% and 3.9% respectively; this was not significantly different from basal level of untreated NCM-460 cells. Upon stimulation with TNF-α, B-GOS significantly attenuated IL-8 and MIP-3α secretion by 43.5% (*p* < 0.001) (Figure 2A) and 52.1% (*p* < 0.05) (Figure 2B) respectively. In the same manner, when NCM-460 cells were prewashed with B-GOS prior to TNF-α stimulation, the secretion of IL8 was significantly reduced by 32% (*p*<0.001) even in the absence of B-GOS (Figure 6). This suggests that constituents of the B-GOS mixture interact with epithelial receptors such as toll-like receptors (TLR) to prevent inflammatory stimulation of the cell.

Similarly upon stimulation with flagellin, B-GOS significantly attenuated IL-8 secretion by 21.5% (p<0.05) (Figure 5). No effect could be observed upon stimulation with IL1β.

To determine if B-GOS is cytotoxic, NCM-460 cells were incubated for 16 hours with or without B-GOS. B-GOS did not affect cell viability as determined by a trypan blue exclusion assay as described in the methods.

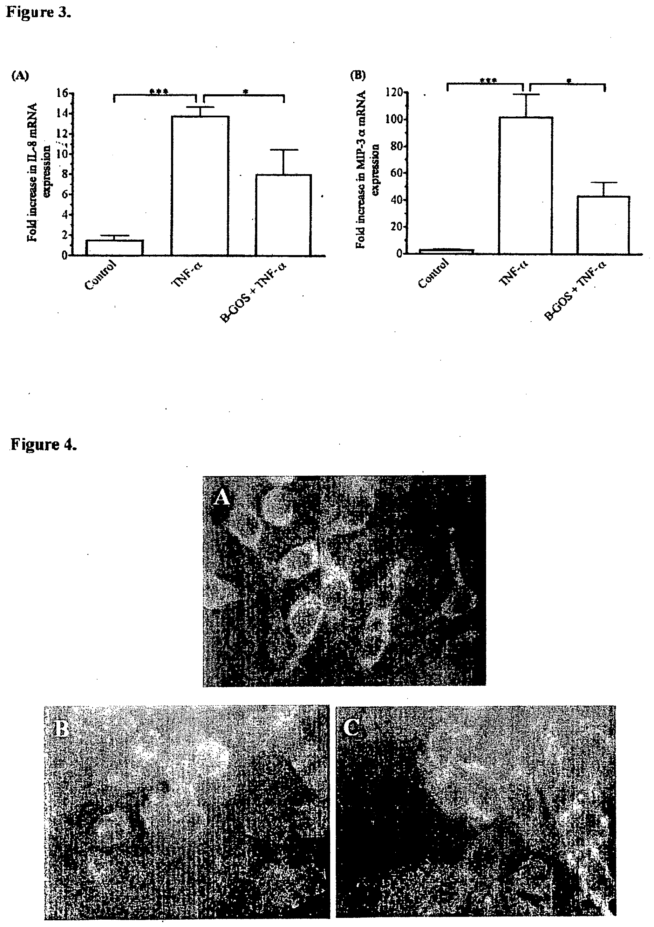

### Effect of galacto-oligosaccharides B-GOS on cytokine expression (Figure 3).

Total RNA of TNF-α treated NCM-460 cells was isolated and assayed for IL-8, MIP-3α and MCP-1 mRNA expression by qRT-PCR. Upon stimulation with TNF-α, IL-8 and MIP-3α mRNA expression was significantly increased by 12.2 fold (*p* < 0.001) (Figure 3A) and 99.4 fold (*p* < 0.001) (Figure 3B) respectively. No change in MCP-1 mRNA expression was observed between any of the treatment (*p* = 0.19) (data not shown). To determine the effect of B-GOS, NCM-460 cells were stimulated with TNF-α in the presence of B-GOS (5 g/L). Galacto-oligosaccharides B-GOS significantly attenuated TNF-α-induced IL-8 and MIP-3α mRNA expression by 5.7 fold (*p* < 0.05) (Figure 3A) and 58.9 fold (*p* < 0.05) (Figure 3B) respectively. The MCP-1 mRNA expression was reduced by B-GOS but did not reach significance (*p* = 0.06) (data not shown).

### Effect of galacto-oligosaccharides B-GOS on NF-κB translocation (Figure 4)

Adult colonic NCM-460 cells were treated with TNF-α (10 ng/mL) and assayed for nuclear translocation of NF-κB p65 protein. In the vehicle treated control cells **(****Figure 4A****)**, staining for NF-κB p65 was predominantly in the cytoplasm and the nucleus was free of p65 protein. NF-κB p65 is clearly translocated into the nuclei after 30 minutes upon stimulation with TNF-α (Figure 4B).

However in the presence of B-GOS, TNF-α-induced NF-κB translocation was partially inhibited at 30 minutes (Figure 4C).

### EXAMPLE 2

### In vivo study of the effect of B-GOS in Dextran Sulphate Sodium induced colitis mouse model

### Material methods

Two groups (n=24 each) of adult C57BL/6 mice (Jackson Laboratories, Bar Harbour, ME, USA), conventionally raised (CR) and bacteria-depleted (BD) mice, were used to induce colitis. All animals were housed within a 12-h light/dark cycle and had access to mouse chow and water ad libitum.

At 6 weeks of age, conventionally colonised mice were housed under conventional conditions with untreated water (CR group) whilst mice in the BD group were fed an antibiotic cocktail in their drinking water for 2 weeks. Kanamycin (8 mg/ml), Gentamicin (0.7 mg/ml), Colistin (34,000 U/ml), Metronidazole (4.3 mg/ml) and Vanacomycin (0.9 mg/ml) comprised the antibiotic cocktail. Concentrations of antibiotics in the water were calculated based on the average water consumed by age group.

At 8 weeks of age, intestinal colitis was induced by feeding 3.5% DSS (Dextran Sulphate Sodium) (MP Biomedicals, Aurora, OH, USA) in drinking water for 5 days in all mice of both groups (CR and BD). At 10 weeks of age, half the mice of each group started receiving Bimuno (5 g/L) for 7 days. At the end of week 10, the animals were euthanized and their colons were harvested for analysis.

### Cytokine measurements

Murine IL-6 and MIP-2 cytokines were analysed by ELISA (Quantikine, R&D Systems, MN, USA) on colon tissue homogenates according to manufacturer's instructions. Briefly, the proximal colons for each group were collected and homogenised with PBS homogenising buffer containing 1% Triton X-100 supplemented with a cocktail of protease inhibitors. The homogenised solutions were centrifuged at 12,000 rpm for 10 min, and the supernatants were separated into aliquots and stored at -70°C.

### Results

The capacity of Bimuno to reduce the injury and inflammation of DSS colitis in both groups of mice (CR and BD) compared to the control mice (no Bimuno administration) was determined.

In conventional DSS-treated mice, IL-6 and MIP-2 secretion was significantly induced by 2.2 (*p* <0.0001) and 8.3 fold (*p* <0.0001) respectively. Bimuno significantly attenuated IL-6 and MIP-2 secretion by 6.6 (*p* <0.0001) and 5.5 fold (*p* <0.0001).

In BD DSS-treated mice, IL-6 and IP-2 secretion was significantly induced by 6.2 (*p* <0.0001) and 27.2 fold (*p* = 0.0005) respectively. Bimuno significantly attenuated IL-6 secretion by 3.6 fold (*p* <0.0001). MIP-2 secretion was reduced by 1.3 fold but this was found to be not significant (*p* = 0.126).

In summary, conventional DSS-treated mice developed colitis compared to the untreated group. DSS-treated conventional mice supplemented with Bimuno had significantly reduced markers of inflammation (IL-6 and MIP-2) and alleviated symptoms of colitis. The same effect was observed in bacteria-depleted DSS-treated mice. This implies that the observed reduction in inflammation due to Bimuno is not only mediated through the microflora. Bimuno has a direct immune-modulatory effect on the intestinal epithelium in DSS colitis.

### EXAMPLE 3

### The effect of fractions of Bimuno galactooligosaccharide mixture on the production of MIP-3α and IL-8 cytokines in the presence of the inflammatory cytokine TNF-α

### Materials and methods

### GOS fractions collection from Bimuno mixture

In order to separate and collect the different fractions of galactooligosaccharides (DP2 deprived of lactose, DP3 and DP>3), the size exclusion chromatography with a Biogel P2 column (100cm x 5cm) (Pharmacia, UK) maintained at room temperature was used. All the samples were eluted at a rate of 2 ml/min with deionised water and were monitored by 132RI detector (Gilson). The lactose was removed from fraction DP2 through the β-galactosidase BIOLACTA FNS provided by TENNOJI-KU (Osaka, Japan). The hydrolysis was conducted at 40°C, at pH 6.4, using 100mM sodium phosphate solution as buffer, and ImM of MgCL₂ as cofactor. The carbohydrate content of each fraction was analysed by ion exclusion and ion exchange chromatography on a RCM-MONOSACCHARIDES Rezex HPLC column equipped with LaChrom RI Detector L-7490 MERCK). The oligosaccharides were eluted in deionised water at 0.5 ml/min at 84.4°C.

### Cell culture

The NCM460 cell line, derived from normal human colon mucosal epithelium, was provided by INCELL CORPORATION LLC and it was maintained in M3Base medium (INCELL CORPORATION LLC) supplemented with 10% [v/v] foetal bovine serum at 37°C in 95% air, 5% CO₂ humidified environment.

### Measurement of cytokine production by NCM460 cultures

NCM460 cells, in concentration of 5 x 10⁵ cells/ml, were plated on 24-well plates and incubated at 37°C in 95% air and 5% CO₂. After 24 hours of incubation, corresponding to 70% of confluence, the cells were treated in triplicate with TNF-α (10ng/ml) (Recombinant Human TNF-α/TNFSF1A - R&D SYSTEMS), fractions of galactooligosaccharides (0.138g/ml of DP2; 0.049g/ml of DP3; 0.041g/ml of DP>3), and mixture of TNF-α and each fraction. The control consisted of cells grown in the medium. The cells were then incubated at 37°C in 95% air, 5% CO₂ for 16 hours. Following the incubation, the supernatant was collected and frozen in aliquots of 200µl, and, before testing, was centrifuged at 400 x g for 5 minutes. The concentration of cytokines (MIP-3α and IL-8) was measured by ELISA assay using the kit QUANTIKINE, provided by R&D Systems (MN, USA).

### Results and discussion

The 3 different fractions, collected using the size exclusion chromatography, had the following amount of galactooligosaccharides:
a) Fraction DP2 contained 90% of galactodisaccharides and the remaining 10% was constituted by monosaccharides (1%), lactose (6%), trisaccharides (2%) and pentaoligosaccharides (1%);
b) Fraction DP3 contained 97% galactotrisaccharide and 3% of DP4 oligosaccharides;
c) Fraction DP>3 contained 96% of galactooligosaccharides with DP4 and DP5, and 4% of galactooligosaccharides with DP3.

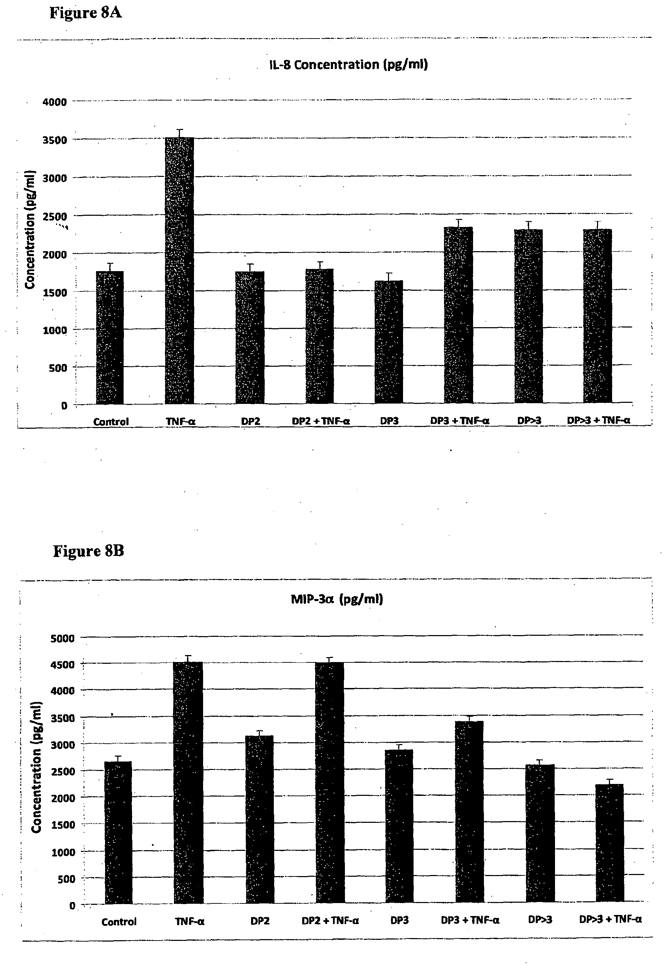

The effect of the fractions on the production of IL-8 and MIP-3α is shown in Figure 8(A) and (B), respectively.

For both experiments, the fraction DP3 and DP>3 decreased the secretion of cytokines:
a) DP3 reduced the IL-8 production by 34% and MIP-3α production by 25% when compared to the TNF-α alone;
b) for the fraction DP>3, the reduction was 35% and 51% for IL-8 and MIP-3α, respectively.

The results for the fraction DP2 were not clear. The reduction for IL-8 secretion was 49%, but this fraction did not have any effect on the MIP-3α production.

### EXAMPLE 4

### The effect of fractions of Bimuno galactooligosaccharide mixture on the production of IL-8 and MIP-3α cytokines in the presence of the inflammatory cytokine TNF-α compared with Bimuno

### Materials and methods

GOS fractions were prepared from Bimuno as described in Example 3.

### Cell cultures

Cell cultures of the NCM460 cell line were obtained from the same source as Example 3 and maintained in the same manner.

### Measurement of cytokine production by NCM460 cultures

NCM460 cells, in concentration of 5 x 10⁵ cells/ml, were plated on 24-well plates and incubated at 37°C in 95% air and 5% CO₂. After 24 hours of incubation, corresponding to 70% of confluence, the cells were treated in triplicate with TNF- α (10ng/ml) (Recombinant Human TNF-α /TNFSF1A - R&D Systems), and mixture of TNF-α and Bimuno (0.1g/ml), TNF-α and the DP=3 fraction (0.1g/ml) of Bimuno or TNF-α and the DP>3 fraction (0.1g/ml) of Bimuno. The control consisted of cells grown in the medium. The cells were then incubated at 37°C in 95% air, 5% CO₂ for 16 hours. Following the incubation, the supernatant was collected and frozen in aliquots of 200µl, and, before testing, was centrifuged at 400 x g for 5 minutes. The concentration of cytokines (MIP-3 and IL-8) was measured by ELISA assay using the kit QUANTIKINE, provided by R&D Systems.

### Results

The 2 different fractions collected using the size exclusion chromatography had the following amount of galactooligosaccharides:
a) Fraction DP3 contained
b) Fraction DP>3 contained

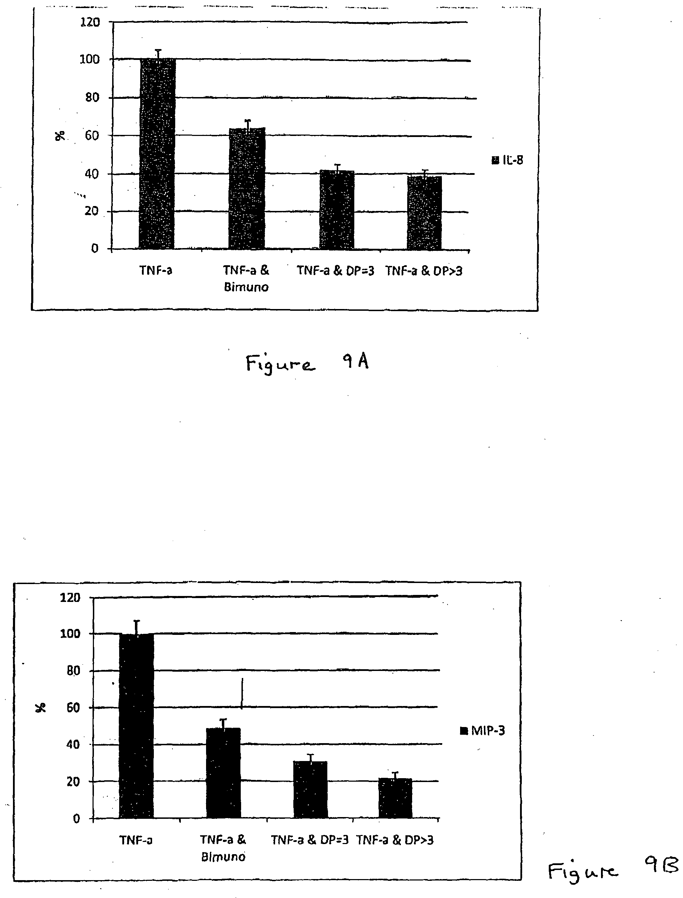

The effect of the galactooligosaccharide fractions and Bimuno on the production of IL-8 and MIP-3α is shown in Figure 9(A) and (B) respectively. From Figures 9(A) and (B) it can be seen that the fraction DP3 reduced the production of IL-8 by 35% more when compared with Bimuno. The reduction in MIP-3α production was 37% greater using the DP3 fraction when compared with Bimuno.

With the fraction DP>3 the reduction in the production of IL-8 compared with Bimuno was 40% greater, and the reduction in MIP-3α production was 55% greater.

### Conclusion

When fractions DP3 and DP>3 are present in amounts equal to the total galactooligosaccharide mixture (Bimuno) the protective effect against inflammation is significantly higher.

## Claims

1. A galactooligosaccharide having a degree of polymerisation of 3 or more, which galactooligosaccharide is selected from the group consisting of trisaccharides Gal (β 1-6) - Gal (β 1-4) - Glc, Gal (β 1-3) - Gal (β 1-4) - Glc, tetrasaccharide Gal (β 1-6) - Gal (β 1-6) - Gal (β 1-4) - Glc and pentasaccharide Gal (β 1-6) - Gal (β 1-6) - Gal (β 1-6) - Gal (β 1-4) - Glc for use in prevention of an intestinal inflammatory disorder.

2. The galactooligosaccharide according to Claim 1 for the use of Claim 1 presented as a powder or a syrup.

## Patentansprüche

1. Galactooligosaccharid, das einen Polymerisationsgrad von 3 oder mehr aufweist, welches Galactooligosaccharid aus der Gruppe ausgewählt ist, die aus Trisacchariden Gal(β1-6)-Gal(β1-4)-Glc, Gal(β1-3)-Gal(β1-4)-Glc, Tetrasaccharid Gal(β1-6)-Gal(β1-6)-Gal(β1-4)-Glc und Pentasaccharid Gal(β1-6)-Gal(β1-6)-Gal(β1-6)-Gal(β1-4)-Glc besteht, für die Verwendung bei der Prävention einer entzündlichen Darmerkrankung.

2. Galactooligosaccharid nach Anspruch 1 für die Verwendung nach Anspruch 1, das als Pulver oder Sirup dargeboten wird.

## Revendications

1. Galacto-oligosaccharide de degré de polymérisation 3 ou plus, lequel galacto-oligosaccharide étant sélectionné dans le groupe consistant en les trisaccharides Gal (β1-6) - Gal (β1-4) - Glc et Gal (β1-3) - Gal (β1-4) - Glc, le tétrasaccharide Gal (β1-6) - Gal (β1-6) - Gal (β1-4) - Glc et le pentasaccharide Gal (β1-6) - Gal (β1-6) - Gal (β1-6) - Gal (β1-4) - Glc pour une utilisation dans la prévention d'une affection intestinale inflammatoire.

2. Galacto-oligosaccharide selon la revendication 1 pour l'utilisation de la revendication 1 qui se présente sous la forme d'une poudre ou d'un sirop.
